# EUROPEAN PATENT SPECIFICATION

(11) **EP 1 602 731 B1**
(45) Date of publication and mention of the grant of the patent: **04.08.2010**
(21) Application number: 03711792.6
(22) Date of filing: 13.03.2003
(51) Int. Cl.: C12Q 1/68

(54) **THE METHOD OF IN VITRO DETECTING ABERRANT gastric DYSPLASIA**
VERFAHREN ZUR IN-VITRO-ERKENNUNG VON ANORMALER Magen-DYSPLASIE
METHODE DE DETECTION IN VITRO D'UNE DYSPLASIE gastrique ABERRANTE

(43) Date of publication of application: 07.12.2005
(73) Proprietor: Beijing Institute for Cancer Research, Beijing 100034 (CN)
(72) Inventor: BAI, Hua, Beijing 100034 (CN); SUN, Yu, Beijing 100036 (CN); ZHOU, Jing, Beijing 100034 (CN); LI, Jiyu, Beijing 100036 (CN); DENG, Dajun, Beijing 100036 (CN)
(74) Representative: von Kreisler Selting Werner
(86) International application number: PCT/CN2003/000180
(87) International publication number: WO 2004/081232

(56) References cited:
- WO-A-03/014388
- WO-A1-01/19845
- WO-A2-01/42493
- WO-A2-01/44504
- EADS C A ET AL: "Epigenetic patterns in the progression of esophageal adenocarcinoma" CANCER RESEARCH, AMERICAN ASSOCIATION FOR CANCER RESEARCH, BALTIMORE, MD, US, vol. 61, no. 8, 15 April 2001 (2001-04-15), pages 3410-3418, XP002982118 ISSN: 0008-5472
- KLUMP B ET AL: "Hypermethylation of the CDKN2/p16 promoter during neoplastic progression in Barrett's esophagus" GASTROENTEROLOGY, ELSEVIER, PHILADELPHIA, PA, US, vol. 115, no. 6, December 1998 (1998-12), pages 1381-1386, XP002305013 ISSN: 0016-5085
- TO KA-FAI ET AL: "Promoter hypermethylation of tumor-related genes in gastric intestinal metaplasia of patients with and without gastric cancer." INTERNATIONAL JOURNAL OF CANCER, vol. 102, no. 6, 20 December 2002 (2002-12-20), pages 623-628, XP002383635 ISSN: 0020-7136
- NIE YAN ET AL: "Detection of multiple gene hypermethylation in the development of esophageal squamous cell carcinoma." CARCINOGENESIS (OXFORD), vol. 23, no. 10, October 2002 (2002-10), pages 1713-1720, XP002383636 ISSN: 0143-3334
- KANG GYEONG HOON ET AL: "CpG island methylation in premalignant stages of gastric carcinoma" CANCER RESEARCH, vol. 61, no. 7, 1 April 2001 (2001-04-01), pages 2847-2851, XP002383637 ISSN: 0008-5472
- BAI HUA ET AL: "p16 hypermethylation during gastric carcinogenesis of Wistar rats by N-methyl-N'-nitro-N-nitrosoguanidine." MUTATION RESEARCH, vol. 535, no. 1, 5 February 2003 (2003-02-05), pages 73-78, XP009067275 ISSN: 0027-5107
- CORREA P: "Human gastric carcinogenesis: a multistep and multifactorial process--First American Cancer Society Award Lecture on Cancer Epidemiology and Prevention." CANCER RESEARCH 15 DEC 1992, vol. 52, no. 24, 15 December 1992 (1992-12-15), pages 6735-6740, ISSN: 0008-5472

## Description

### TECHNICAL FIELD OF THE INVENTION

The present invention relates to detection of malignant potential of dysplasia. The present invention provides a method to measure the potential of malignant progression or transformation of dysplasia, a precancerous lesion of carcinomas, by detection of methylation of *p16* CpG islands in DNA of dysplasia lesions. The present invention also provides the artificial DNA sequences corresponding to *p16* CpG islands that are used as biomarkers for patients with dysplasia.

### BACKGROUND OF THE INVENTION

Cancer is one of the major causes of human death. Detection, diagnosis, and treatment at early stage of carcinogenesis are very efficient strategies against cancer. Epithelial dysplasia is a precancerous lesion that is observed frequently in various organs such as oral cavity, esophagus, stomach, intestine, liver, etc. It is well known that some dysplasia lesions will progress malignantly. Therefore, the traditional word "dysplasia" is also called "non-invasive neoplasia" in Padova international classification. However, dysplasia lesions are not always progressive. In fact, they are persistent or regressive in most of cases.

Current diagnosis for dysplasia is primarily based on morphologic criteria. Dysplasia lesions are classified as low-grade dysplasia and high-grade dysplasia according to those criteria. The higher grade, the higher risk. In a 5-year fellow-up screening, about 3% of low-grade dysplasia transformed malignantly and 7%, high-grade ones (You WC, Li JY, Blot WJ, Chang YS, Jin ML, Gail MH, Zhang L, Liu WD, Ma JL, Hu YR, Mark SD, Correa P, Fraumeni JF, Xu GW. Evolution of precancerous lesions in a rural Chinese population at high risk of gastric cancer. Int J Cancer 1999; 83: 615-619). It is virtually impossible to identify malignant potential of low-grade dysplasia lesions on histopathologic grounds alone.

The risk of stomach cancer for patients with high-grade gastric dysplasia is up to 100-fold of that for patients with normal gastric mucosa. This results two contrast consequences. In one hand, patients with gastric dysplasia are often treated with measures used to treat patients with cancer. For example, stomach resection is administrated in some cases. These led to over-treatment among 90% of patients with dysplasia that does not progress. Therefore, huge amount of medical resources are wasted and patients are hurt unnecessary. In another hand, some patients lose the best chance for them to get the right management in time, because of their poor social-economic status or ignorance of the high risk of cancer. Thus, predicting the malignant potential of this lesion is eagerly awaited. Such a predicating method will not only save many patients' life, but also bring important economic benefit.

Understanding of carcinogenesis is advanced significantly and comprehensively by recent developments of molecular biology. It is observed that lost of function of tumor suppressor genes could result from structural changes of DNA sequences and from changes not related with DNA sequences. The structural changes of a target gene are numerous, including point mutation, deletion, insertion, translocation, and amplification. Not all structural changes result in alteration of gene function. The non-structural changes are mainly epigenetic changes, including alteration of methylation pattern of CpG islands, modifications of histones, and chromatin remodeling. Hypermethylation of CpG islands silences gene transcription completely. Hence, it is simpler and more meaningful to detect CpG methylation than to detect structural changes of a target gene for clinical practice. Epigenetic changes play an important role in carcinogenesis.

Methylation-silence of tumor suppressor gene(s) might result in formation of malignant potential of few cells in precancerous lesions such as dysplasia. Detection of the cells with the CpG methylation-silenced gene(s) might be very valuable for predication of malignant potential of dysplasia. Reversed transcriptional-PCR assay is useful to detect mRNA expression of genes. Immunostaining and Western blot are regular assays for protein products of gene expressions. However, results of these assays show gene expression status presenting in the majority of cell populations. Thus, they are not suitable for detection of abnormal silence of gene expression presenting only in a few cells among cell populations when majority of them express the target gene normally.

Some methylation-silenced genes were used as biomarkers for predication of malignant transformation successfully. For example, malignant progression of myelodysplastic syndromes was associated with methylation of *p15* (Uchida T, Kinoshita T, Nagai H, Nakahara Y, Saito H, Hotta T, Murate T. Hypermethylation of the p15INK4B gene in myelodysplastic syndromes. Blood 1997; 90: 1403-1409; Quesnel B, Guillerm G, Vereecque R, Wattel E, Preudhomme C, Bauters F, Vanrumbeke M, Fenaux P. Methylation of the p15(INK4b) gene in myelodysplastic syndromes is frequent and acquired during disease progression. Blood 1998; 91: 2985-2990). The majority of microsatellite unstable sporadic colon, endometrium, stomach cancers were correlated with transcription silence of *hMLH1* by CpG methylation (Hawkins N, Norrie M, Cheong K, Mokany E, Ku SL, Meagher A, O'Connor T, Ward R. CpG island methylation in sporadic colorectal cancers and its relationship to microsatellite instability. Gastroenterology 2002; 122:1376-1387; Esteller M, Catasus L, Matias-Guiu X, Mutter GL, Prat J, Baylin SB, Herman JG. hMLH1 promoter hypermethylation is an early event in human endometrial tumorigenesis. Am J Pathol 1999; 155:1767-1772; Guo RJ, Arai H, Kitayama Y, Igarashi H, Hemmi H, Arai T, Hanai H, Sugimura H. Microsatellite instability of papillary subtype of human gastric adenocarcinoma and hMLH1 promoter hypermethylation in the surrounding mucosa. Pathol Int 2001; 51:240-247). Detections of hypermethylation of *p15* and *hMLH1* are clinical assays now. Methylation of *p16* was detectable in serum DNA samples from patients with cancer (Esteller M, Sanchez-Cespedes M, Rosell R, Sidransky D, Baylin SB, Herman JG. Detection of aberrant promoter hypermethylation of tumor suppressor genes in serum DNA from non-small cell lung cancer patients. Cancer Res 1999; 59:67-70 Erratum in: Cancer Res 1999; 59:3853; Wong IH, Lo YM, Zhang J, Liew CT, Ng MH, Wong N, Lai PB, Lau WY, Hjelm NM, Johnson PJ. Detection of aberrant p16 methylation in the plasma and serum of liver cancer patients. Cancer Res 1999; 59:71-73; Sanchez-Cespedes M, Esteller M, Wu L, Nawroz-Danish H, Yoo GH, Koch WM, Jen J, Herman JG, Sidransky D. Gene promoter hypermethylation in tumors and serum of head and neck cancer patients. Cancer Res 2000; 60:892-895). There is no report that malignant potential of precancerous lesions such as dysplasia could be predicated by detection of methylation of *p16* CpG islands.

WO 03/014388A disclosed a large number of bisulphate-modihed DNA sequences, including p16, in the supposed optimal cases in which all CpG sites in the sequences are fully methylated or not methylated at all and all of the unmethylated cytosines are modified. They used the supposed sequences to design oligonucleotide sets for detection of methylation of a large number of CpG islands.

The article "Epigenetic patterns in the progression of esophageal adenocarcinoma", Cindy A. Eads et al. Cancer Research 61, 3410-3418, April 15, 2001, detected methylation status of 20 genes, including CDKN2A/p16, in 84 tissue samples from 51 patients with Barrett's esophagus and/or associated adenocarcinomas and found that those genes could be sub-classified into seven classes. They also found that normal and metaplastic tissues from patient with dysplasia or cancer had a significantly higher incidence of hypermethylation than similar tissues from patients with no further progression of their disease. However, they did not observe any significant difference between dysplasia and adenocarcinoma for any of those genes or their combinations.

The article "Hypermethylation of the CDKN2/p16 promoter during neoplastic progression in Barrett's esophagus", Bodo Klump et al., Gastroenterology 1998;115: 1381-1386, detected p16 methylation in 95 specimens from 14 patients with Barrett's esophagus and found that p16 methylation was detectable in 9 of the 10 patients displayed dysplasia at some time during surveillance, but in none of the patients that did not display dysplasia. Their data suggested that p16 methylation might be an early event before dysplasia in Barrett's esophagus. This publication did not investigate the relationship between p16 methylation and malignant potential of epithelial dysplasia.

The article "Promoter hypermethylation of tumor-related genes in gastric intestinal metaplasia of patients with and without gastric cancer", Ka-Fai To et al., Int. J. Cancer: 102, 623-628 (2002), detected methylation status of 9 genes, including p16, in gastric carcinoma and intestinal metaplasia (IM) samples. They found that the mean number of methylated genes in cancer was higher than that in IM, and that the methylation pattern in IM adjacent gastric carcinoma was similar to that in IM from non-cancerous patients. This publication did not investigate the relationship between p16 methylation and malignant potential of epithelial dysplasia.

The article "Detection of multiple gene hypermethylation in the development of esophageal squamous cell carcinoma", Yan Nie et al., Carcinogenesis vol.23. no.10 pp. 1713-1720, 2002, analyzed methylation status of 10 genes, including p16, in esophagus samples with various pathological lesions and found the mean number of methylated genes in esophagus carcinoma was higher than that in their adjacent lesions such as basal cell hyperplasia, dysplasia. This publication did not investigate the relationship between p16 methylation and malignant potential of epithelial dysplasia.

The article "CpG island methylation in premalignant stages of gastric carcinoma", Gyeong Hoon Kang et al., Carcer Research 61. 2847-2851, April 1, 2001, analyzed the methylation patterns of 5 genes, including p16, in human gastric carcinoma and precancerous lesion samples, and found a marked increase in methylated genes from non-metaplastic mucosa to intestinal metaplasia as well as from premalignant lesions to carcinomas. This publication did not investigate the relationship between p16 methylation and malignant potential of epithelial dysplasia.

### DETAILED DESCRIPTION OF THE INVENTION

The present invention provides for an in vitro detection assay for malignant potential of dysplasia. The present invention is developed technically according to the following hypothesis and confirmation investigation.

HYPOTHESIS: *p16* is an important tumor suppressor gene including a CpG island around transcriptional start site. Transcription of *p16* is regulated by the methylation pattern of the CpG island. That *p16* CpG island is unmethylated enable regular expression of *p16* in normal cells. Aberrant methylation of the CpG island silences *p16* transcription. Such abnormal *p16* methylation was frequent in many kinds of tumors. It was reported that *p16* methylation presented in 40% of gastric carcinomas (Kang GH, Shim YH, Jung HY, Kim WH, Ro JY, Rhyu MG. CpG island methylation in premalignant stages of gastric carcinoma. Cancer Res 2001; 61: 2847-2851). Our dynamic study on a rat model for gastric carcinogenesis showed that *p16* methylation frequency was correlated with the severity of gastric pathologic lesions positively. For instance, *p16* methylation was found in 2.7% of normal gastric epithelium (n=36), 16.7% of chronic atrophy gastritis (n=24), 37.5% of dysplasia (n=24), 67.4% of gastric adenoma (n=43), and 85.2% of gastric carcinoma (n=27). (Bai H, Gu LK, Zhou J, Deng DJ. p16 hypermethylation during gastric carcinogenesis of Wistar rats by N-methyl-N'-nitro-N-nitrosoguanidine. Mutat Res 2003; 535: 73-78). These results suggested that *p16* methylation might be an early event whose accumulation will finally lead to gastric carcinogenesis. Hence, we hypothesize that *p16* methylation might be a valuable marker for detection of malignant potential of precancerous lesions.

CONFIRMATION: To validate above hypothesis, we carried out a nested case-control study on a 5-year follow-up endoscopic screening in a high-risk population. Aberrant *p16* methylation was observed in 5 of 21 samples of dysplasia progressed to gastric carcinoma, but none of 21 ones without progression (p=0.048, 2-sides). This result proves that presence of the methylated *p16* CpG island correlates with malignant progression of gastric dysplasia positively and significantly. In another word, detection of *p16* methylation may be a precise assay for predication of malignant potential of -gastric dysplasia. Presence of *p16-*methylated cells in gastric dysplasia lesion is a strong diagnostic and prognostic indicator of malignant transformation of the lesions.

The present invention provides the assay for detection of malignant potential of gastric dysplasia composing the following steps: A). Extraction of genomic DNA of samples of target tissues of body fluids from patients; B). Detection of methylation of *p16* CpG island; C). Evaluation of malignant potential of testing tissue.

The present invention for in vitro predication of malignant potential of gastric dysplasia within methylation of *p16* CpG island is detected with methylation-specific PCR (MSP, refers to the art-recognized methylation assay described by Herman JG, Graff JR, Myohanen S, Nelkin BD, Baylin Sb. Methylation-specific PCR: a novel PCR assay for methylation status of CpG islands. Proc. Natl. Acad. Sci. USA 1996; 93: 9821-9826, and by US Patent No. 5,786,146). A set of positive control and negative control samples are also used as quality controls.

For the present invention a pair of methylated sequence specific MSP primers are designed and synthesized which are complementary to SEQ ID NO:1 and SEQ ID NO:3, and a pair of unmethylated sequence specific MSP primers are designed and synthesized, which are complementary to SEQ ID NO: 2 and SEQ ID NO: 4. The mentioned chemical modification of DNA means deamination of unmethylated cytosines by chemicals such as sodium bisulfite, which converts only unmethylated cytosine to uracil (U) and does not convert methylated cytosines.

The present disclosure further provides a set of artificial DNA sequences, SEQ ID NO: 1, SEQ ID NO: 2, SEQ ID NO: 3, and SEQ ID NO: 4, which are used to design MSP primers for detection of *p16* methylation.

The present invention also provides the following diagnostic criteria for evaluation of malignant potential of testing lesions according to the results obtained by the present assay.
A). When MSP products of methylated *p16* CpG island can not be amplified from the chemical modified unmethylated *p16* template (negative control) by the methylated-sequence -specific primers, AND when MSP products of methylated *p16* CpG island CAN be amplified from the chemical modified templates from testing lesion in the same time under the same conditions, this lesion is highly malignant.
B). When MSP products of methylated *p16* CpG island can be amplified from the chemical modified methylated *p16* template (positive control) by the methylated-sequence-specific primers, AND when MSP products of methylated *p16* CpG island CANNOT be amplified from the chemical modified templates from testing lesion, AND when MSP products of unmethylated *p16* CpG island can be amplified from the same testing templates, but can not be amplified form the positive control templates, this lesion can not exclude from malignant one.
C). When both MSP products of methylated and unmethylated *p16* CpG island CANNOT be amplified from the chemical modified templates from testing lesion by the methylated- and unmethylated-sequence-specific primers, respectively, repeat assay should be carried out till MSP products of methylated OR unmethylated *p16* CpG island can be amplified from the testing templates.

The advantages of the present invention include: A). Prove firstly that the methylated *p16* CpG island can be used as biomarker for precise predication of malignant potential of gastric dysplasia. Very limited number of abnormal cells with methylation-silenced *p16* in dysplasia lesion can be detected sensitively by MSP assay. B). High specificity and very early predication. The malignant potential of dysplasia can be detected as early as 5-year before dysplasia lesion progresses to carcinoma.

Detection of hypermethylation of *hMLH1* CpG island is used clinically for detection of malignant potential of colon polyp to prevent colon cancer. The methylation-silenced *p16* might be the second discovered epigenetic biomarker for predication of malignant potential of precancerous lesions. Prevalence of methylated *p16* CpG island is 20%∼40% of various cancers, which is much higher than that of methylation-silenced *hMLH1* (5%∼10%). Therefore, it is reasonable to expect a much wider application of the present invention clinically.

To help further understanding of the present invention, the following figures, figure legends, and example are intended to illustrate but not limit the invention.

### BRIEF DESCRIPTION OF THE FIGURES

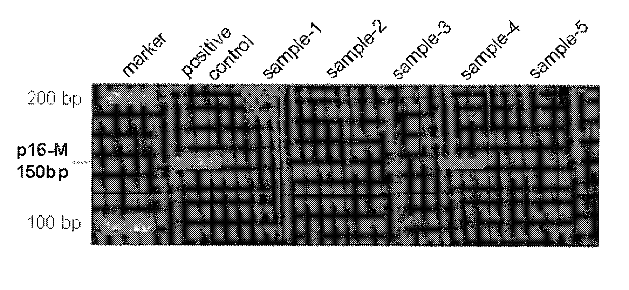

Figure 1 shows the result of MSP products of methylated *p16* CpG islands from the chemical modified genomic DNA samples extracted from paraffin-embedded tissue blocks.
   "SEQ ID NO: 1" is the artificial sequence of antisense strand of methylated *p16* CpG island with chemical modification. All uracil (U) in the sequence can be replaced by thymidine (t) in PCR products if dTTP is used.
   "SEQ ID NO: 2" is the artificial sequence of antisense strand of unmethylated *p16* CpG island with chemical modification. All uracil (U) in the sequence can be replaced by thymidine (t) in PCR products if dTTP is used.
   "SEQ ID NO: 3" is the artificial sequence of sense strand of methylated *p16* CpG island with chemical modification. All uracil (U) in the sequence can be replaced by thymidine (t) in PCR products if dTTP is used.
   "SEQ ID NO: 4" is the artificial sequence of sense strand of unmethylated *p16* CpG island with chemical modification. All uracil (U) in the sequence can be replaced by thymidine (t) in PCR products if dTTP is used.

### EXAMPLE

In Vitro Predication of Malignant Potential of Gastric Dysplasia by Detection of Modified Sequence of Methylated *p16* CpG Islands
1. Specimens and Objects: Endoscopic gastric biopsies with low-grade dysplasia at baseline either progressed to gastric carcinoma or persisted in dysplasia at the corresponding sites during the 5-year follow-up were selected for detection of *p16* promoter methylation. All of biopsy samples (n=21) of dysplasia progressed to gastric carcinoma was used if sections were available from the paraffin block. Equal number of samples (*n*=21) of dysplasia persisted in dysplasia was selected from the tissue block archive, according to the pathological grade, sampling site, age, and sex. Distilled water and genomic DNA of human gastric carcinoma (*p16* unmethylated, by bisulfite-sequencing) or lymphocytes were used as template for negative control. Genomic DNA of *p16*-methylated human gastric carcinoma (*p16* methylated, by bisulfite-sequencing) or colon cancer cell line RKO was used as positive control.
2. Harvesting Cells: All sections from the margin of the gastric tissue biopsies embedded in paraffin were collected into 1.5 ml microcentrifuge tube from each sample. The collected tissue sections were dewaxed by xylene, and rehydrated with graded ethanol before extraction of DNA samples.
3. Extraction of Genomic DNA: The collected sections were mixed with 300 µl of lysis buffer (10 mM Tris.Cl/pH7.6, 10 mM NaCl, 10 mM EDTA, and 0.5% SDS) and 10 µl of proteinase K solution (20 mg/ml), digested at 37°C or 55°C overnight. Then, the digestion was incubated at 100°C to inactivate proteinase K. About 10 ng∼50 ng genomic DNA was extracted from each sample.
4. Chemical Modification of Unmethylated Cytosines:
   a) Add 50 µl of distilled sterile water into 1.5 ml microcentrifuge tube with above extracted genomic DNA.
   b) To denature double strands DNA, add 5.5 µl of 3M NaOH, mix, incubate at 50°C ∼55°C for 15 min.
   c) Add 30 µl of fresh 10 mM hydroquinone, mix.
   d) To sulfite DNA, add 520 µl of fresh 1.5 M Na₂S₂O₅ (equal to 3M NaHSO₃), mix. Cover the top of reaction with 200 µl of mineral oil to prevent evaporation of reaction. Incubate at 50°C for 16h to deamine unmethylated cytosines.
   e) Remove mineral oil. Purify the modified DNA with Wizard DNA Clean-Up System (Promega A7280) as kit instruction: Attach syringe to minicolumn and insert the tip of column into the vacuum manifold, one set for one sample. Add 1 ml of resin solution suspended at 30°C into microcentrifuge tube, pipette resin/DNA mix down and up, transfer the mix into column-syringe assembly, remain 5 min. Apply a vacuum to draw the solution through the column. DNA-resin binding complex was remained on the column. Break the vacuum. To wash the complexes in the column, add 2 ml of 80% isopropanol to the syringe, and reapply a vacuum to draw the solution through the column. Dry the DNA-resin complexes by continuing to draw a vacuum for 30sec after the solution has been pulled through the column, transfer the column to a 1.5 ml microcentrifuge tube. Centrifuge the column at maximum speed (10,000g) for 20 sec to remove any residual isopropanol. Transfer the column to a new microcentrifuge tube. Apply 50 µl of pre-warmed (80°C) water and remain 5 min at 80°C. Centrifuge the column for 20 sec at maximum speed (10,000g) to elute the bound DNA. Reapply 50 µl of pre-warmed (80°C) water and remain 10 min at room temperature (RT). Centrifuge again. Remove and discard the column.
   f) To complete the modification, add 11 µl of 3M NaOH, mix, remain 5min at RT.
   g) To precipitate DNA and remove NaOH, add 166 µl of 5M NaOAC and 750 µl of 100% cold ethanol, mix, store at -20°C for 2h. Centrifuge at 10,000g for 30 min. Discard solution. Add 200 µl of 80% cold ethanol to wash DNA. Centrifuge again. Discard solution.
   h) Resuspend the DNA in 3∼6 µl of sterile water or TE buffer. Use immediately or store at -20°C.
5. Design PCR primers: According to the modified sequences (SEQ ID NO: 1, SEQ ID NO: 2, SEQ ID NO: 3, and SEQ ID NO: 4) of methylated and unmethylated *p16* CpG islands, design methylated-sequence-specific primers (sense, 5'-ttattagagg gtgggCgga tCgC-3'; antisense, 5'-GaccccGaac cGcGaccGta a-3') and unmethylated-sequence-specific primers (sense, 5'-ttattagagg gtggggTgga tTgT-3'; antisense, 5'-cAaccccAaa ccAcAaccAt aa-3'), respectively. These regions of the artificial sequences containing frequent uracils (U) and CpG or UpG are selected for the complemental sequence of 3' end of each primer.
6. PCR Amplification: the modified templates of methylated *p16* CpG islands were amplified by hot-start PCR. PCR products of the target sequence could be displayed by any chromatography technologies such as agarose gel, PAGE gel, HPLC, etc. PCR products of the methylated templates were also confirmed by sequencing. If PCR products of the methylated templates cannot be amplified from the testing samples, PCR products of the unmethylated templates should be amplified further to exclude the failures of DNA extraction, modification, and purification. Both positive control and negative control samples are used to exclude possible contamination or failures of amplification.
7. Results: Aberrant *p16* methylation was observed in 5 of 21 samples of dysplasia progressed to gastric carcinoma, but none of 21 ones without progression (*p*=0.048, 2-sides). Sequencing results confirmed that all CpG sites were methylated in the analyzed sequence from these five *p16-*methylated cases. Unmethylated *p16* CpG islands were detected in all of the samples without *p16* methylation.
8. Conclusions: The present assay can predicate malignant potential of gastric dysplasia specifically. Aberrant *p16* methylation was not observed in all samples of dysplasia without progression (Specificity, 100%). The sensitivity for detection of malignant potential of all samples of dysplasia progressed to gastric carcinomas is only 24%. However, the sensitivity for detection of malignant potential of these samples of *p16*-methylated dysplasia is very high, because all 5 patients with *p16-*methylated gastric dysplasia progress to gastric carcinomas at the sampling sites of stomach five year later (Sensitivity, 100%).
9. Alternative Protocols: aberrant *p16* methylation are also detectable in genomic DNA of cells collected from fasting gastric juice.

All the persons working on these fields understand that the malignant potential of dysplasia can be detected by any methods that can be used for detection of methylation of *p16* CpG islands. The example presented above is intended to illustrate but not limit the invention.

## Claims

1. A method for in vitro detection of malignant potential of gastric dysplasia, comprising:
(a) extraction of genomic DNA from cells from samples from gastric dysplasia;
(b) detection of methylation state of *p16 CpG* islands;
(c) evaluation of malignant potential of the samples based upon presence of methylated *p16 CpG* islands.

2. A method for in vitro detection of malignant potential of dysplasia of claim 1 wherein the methylation state of *p16 CpG* islands is analyzed by methylation-specific PCR (MSP).

3. A method for in vitro detection of malignant potential of dysplasia of claim 2 wherein methylated-sequence specific primers are complementary to any part of the artificial sequence SEQ ID NO: 1 or SEQ ID NO: 3, or wherein unmethylated-sequence specific primers are complementary to any part of the artificial sequence SEQ ID NO: 2 or SEQ ID NO:4.

## Patentansprüche

1. Verfahren zum In-vitro-Nachweis des malignen Potentials einer Dysplasie der Magenschleimhaut, umfassend:
(a) Extraktion der genomischen DNA von Zellen aus Proben einer Dysplasie der Magenschleimhaut;
(b) Nachweis des Methylierungszustands von p16-CpG-Inseln;
(c) Bewertung des malignen Potentials der Proben anhand der Anwesenheit von methylierten p16-CpG-Inseln.

2. Verfahren zum In-vitro-Nachweis des malignen Potentials einer Dysplasie gemäß Anspruch 1, wobei der Methylierungszustand der p16-CpG-Inseln durch methylierungsspezifische PCR (MSP) analysiert wird.

3. Verfahren zum In-vitro-Nachweis des malignen Potentials einer Dysplasie gemäß Anspruch 2, wobei für eine methylierte Sequenz spezifische Primer komplementär zu irgendeinem Teil der künstlichen Sequenz SEQ ID Nr. 1 oder SEQ ID Nr. 3 sind oder wobei für eine unmethylierte Sequenz spezifische Primer komplementär zu irgendeinem Teil der künstlichen Sequenz SEQ ID Nr. 2 oder SEQ ID Nr. 4 sind.

## Revendications

1. Procédé de détection *in vitro* du potentiel de malignité d'une dysplasie gastrique, comprenant :
(a) l'extraction de l'ADN génomique de cellules issues d'échantillons de dysplasie gastrique ;
(b) la détection de l'état de méthylation des îlots *CpG* de *p16* ;
(c) l'évaluation du potentiel de malignité des échantillons sur la base de la présence d'îlots *CpG* de *p16* méthylés.

2. Procédé de détection *in vitro* du potentiel de malignité d'une dysplasie selon la revendication 1, dans lequel l'état de méthylation des îlots *CpG* de *p16* est analysé par PCR spécifique de la méthylation (MSP).

3. Procédé de détection *in vitro* du potentiel de malignité d'une dysplasie selon la revendication 2, dans lequel des amorces spécifiques de la séquence méthylée sont complémentaires de toute partie de la séquence artificielle SEQ ID N° 1 ou SEQ ID N° 3, ou dans lequel des amorces spécifiques de la séquence non méthylée sont complémentaires de toute partie de la séquence artificielle SEQ ID N° 2 ou SEQ ID N° 4.


### SEQUENCE TABLE

<110> Beijing Institute for Cancer Research
<120> THE METHOD OF IN VITRO DETECTING ABERRANT DYSPLASIA, AND THE ARTIFICIAL NUCLEOTIDES BEING USED
<130> CNB1U03000217
<160> 4
<210> 1
   <211> 359
   <212> DNA
   <213> Artificial sequence
<220>
   <223> Artificial sequence
<400> 1
   agaggagggg ttggttggtt attagagggt ggggcggatc gcgtgcgttc ggcggttgcg  60
   gagaggggga gagtaggtag cgggcggcgg ggagtagtat ggagtcggcg gcggggagta 120
   gtatggagtt ttcggttgat tggttggtta cggtcgcggt tcggggtcgg gtagaggagg 180
   tgcgggcgtt gttggaggcg ggggcgttgt ttaacgtatc gaatagttac ggtcggaggt 240
   cgatttaggt gggtagaggg tttgtagcgg gagtagggga tggcgggcga ttttggagga 300
   cgaagtttgt aggggaattg gaattaggta gcgtttcgat ttttcggaaa aaggggagg  359
<210> 2
   <211> 359
   <212> DNA
   <213> Artificial sequence
<220>
   <223> Artificial sequence
<400> 2
   agaggagggg ttggttggtt attagagggt ggggtggatt gtgtgtgttt ggtggttgtg  60
   gagaggggga gagtaggtag tgggtggtgg ggagtagtat ggagttggtg gtggggagta 120
   gtatggagtt tttggttgat tggttggtta tggttgtggt ttggggttgg gtagaggagg 180
   tgtgggtgtt gttggaggtg ggggtgttgt ttaatgtatt gaatagttat ggttggaggt 240
   tgatttaggt gggtagaggg tttgtagtgg gagtagggga tggtgggtga ttttggagga 300
   tgaagtttgt aggggaattg gaattaggta gtgttttgat tttttggaaa aaggggagg  359
<210> 3
   <211> 359
   <212> DNA
   <213> Artificial sequence
<220>
   <223> Artificial sequence
<400> 3
   tttttttttt tttcggagaa tcgaagcgtt atttgatttt aatttttttg taaatttcgt  60
   tttttagagt cgttcgttat tttttgtttt cgttgtagat tttttattta tttggatcgg 120
   ttttcgatcg taattattcg gtgcgttggg tagcgttttc gtttttagta gcgttcgtat 180
   tttttttatt cgatttcggg tcgcggtcgt ggttagttag ttagtcgaag gttttatgtt 240
   gtttttcgtc gtcggtttta tgttgttttt cgtcgttcgt tgtttgtttt tttttttttc 300
   gtagtcgtcg agcgtacgcg gttcgtttta ttttttggtg attagttagt ttttttttt  359
<210> 4
   <211> 359
   <212> DNA
   <213> Artificial sequence
<220>
   <223> Artificial sequence
<400> 4
   tttttttttt ttttggagaa ttgaagtgtt atttgatttt aatttttttg taaattttgt  60
   tttttagagt tgtttgttat tttttgtttt tgttgtagat tttttattta tttggattgg 120
   tttttgattg taattatttg gtgtgttggg tagtgttttt gtttttagta gtgtttgtat 180
   tttttttatt tgatttcggg ttgtggttgt ggttagttag ttagttgaag gttttatgtt 240
   gttttttgtt gttggtttta tgttgttttt tgttgtttgt tgtttgtttt tttttttttt 300
   gtagttgttg agtgtatgtg gtttgtttta ttttttggtg attagttagt ttttttttt  359